# EUROPEAN PATENT APPLICATION

(11) **EP 2 407 930 A1**
(43) Date of publication of application: **18.01.2012**
(21) Application number: 10750495.3
(22) Date of filing: 22.02.2010
(51) Int. Cl.: G06T 7/00, A61B 5/117, G06T 1/00

(54) **FACE AUTHENTIFICATION DEVICE, PERSON IMAGE SEARCH SYSTEM, FACE AUTHENTIFICATION DEVICE CONTROL PROGRAM, COMPUTER READABLE RECORDING MEDIUM, AND METHOD OF CONTROLLING FACE AUTHENTIFICATION DEVICE**

(30) Priority: 13.03.2009 JP 2009062041
(71) Applicant: Omron Corporation, Kyoto-shi, Kyoto 600-8530 (JP)
(72) Inventor: HOSOI, Satoshi, Kyoto-shi, kyoto 600-8530 (JP)
(74) Representative: Giovannini, Francesca
(86) International application number: PCT/JP2010/001140
(87) International publication number: WO 2010/103736

(57) **Abstract**

The present invention further improves an accuracy of face authentication procedure without need for any special video equipment and others. In a face authentication apparatus (100) including a face verifying section (56) that determines degrees of similarity between incoming input face image data and a plurality of registered facial characteristics information items that have been preregistered, and then extracts the registered facial characteristics information item having the degree of similarity to the input face image data of not less than a predetermined value, the registered facial information items are respectively registered together with registered attribute information items on persons related to the registered facial characteristics information items, and the face authentication apparatus (100) further includes: a face authentication section (50) that identifies which of the plurality of registered facial characteristics information items that have been extracted by the face verifying section (56) is the registered facial characteristics information item containing a person related to the input face image data, the face authentication section (50) includes: a gender/age estimating section (54) that estimates an attribute of the person related to the input face image data and then outputs an attribute information item; and a gender/age comparing section (58) that makes comparison between the attribute information item thus outputted and the registered attribute information items corresponding to the plurality of registered facial characteristics information items, whereby the registered facial information item is identified on a basis of a result of the comparison.

## Description

### Technical Field

The present invention relates to a face authentication apparatus which performs face authentication procedure with respect to image data representing a face of a person, a person image search system, a face authentication apparatus control program, a computer-readable recording medium, and a method of controlling the face authentication apparatus.

### Background Art

Conventionally, in the field of face authentication technology, the following technique is known. That is, a person's face is detected from an image targeted for search (e.g. Patent Literature 1), and the face thus detected is then verified on the basis of face authentication information such as the amount of characteristics that have been preregistered, so that authentication is made to identify whose face the face contained in that image is (e.g. Patent Literature 2).

Further, there has been suggested the following method (Patent Literature 3) aiming for improving an accuracy of face authentication procedure. That is, a distance between tails of eyes of a subject is measured by means of a twin-lens stereo camera, so that authentication is made on the basis of the distance thus measured. Thus, there has been suggested the face authentication technology using physical characteristics of the subject, such as a distance between tails of eyes.

In addition, there has been suggested a technique for speeding up the face authentication procedure. The technique for speed-up is such that face authentication information items to be used in the face authentication procedure are previously narrowed down by attribute information, such as gender, age, and generation, and face authentication is then performed (e.g. Patent Literatures 4 and 5). Further, research and development has been pursued on a technique of estimating the attribute information, such as gender, age, and generation, from an face image with a high degree of accuracy (e.g. Non-Patent Literature 1).

### Citation List

### Patent Literatures

Patent Literature 1
   Japanese Patent Application Publication, Tokukai, No. 2005-327242 A (Publication Date: November 24, 2005)
Patent Literature 2
   Japanese Patent Application Publication, Tokukai, No. 2007-128262 A (Publication Date: May 24, 2007)
Patent Literature 3
   Japanese Patent Application Publication, Tokukai, No. 2007-114931 A (Publication Date: May 10, 2007)
Patent Literature 4
   Japanese Patent Application Publication, Tokukai, No. 2008-158679 A (Publication Date: July 10, 2008)
Patent Literature 5
   Japanese Patent Application Publication, Tokukai, No. 2006-146413 A (Publication Date: June 8, 2006)

### Non-Patent Literatures

### Non-Patent Literature 1

Erina TAKIGAWA, Satoshi HOSOI, "Automatic gender and generation estimation from face image", OMRON TECHNICS, March in 2003, Vol. 43, 1st issue (145th issues in total), pp. 37-41

### Summary of Invention

### Technical Problem

However, the technique of aiming for improving an accuracy of the face authentication procedure by a method of measuring a distance between tails of eyes of a subject by means of a twin-lens stereo camera requires the twin-lens stereo camera to be provided. This method involves measurement of a distance between tails of eyes and cannot be used for face image search targeted for images on which faces have already captured. This is because measurement of a distance between tails of eyes of a subject that shows up on an image lacks precision due to change in angle of a face and other conditions.

Further, the method of narrowing down authentication information items to be used in the face authentication procedure by using attribute information, and then performing face authentication allows for speeding up the face image search. However, this method does not contribute to the improvement of an accuracy of face authentication itself. If anything, this method tends to decrease an accuracy of the face authentication procedure for the following reason. If the authentication information items are incorrectly narrowed down, the authentication information items to be used in the face authentication falls outside the scope of a search. This may cause a failure to arrive at the image to be outputted as a result of the authentication.

The present invention has been attained in view of the foregoing problems and an object of the present invention is to realize: a face authentication apparatus which enables further improvement of an accuracy of the face authentication procedure without need for any special video equipment (e.g. twin-lens stereo camera) and others; a person image search system; a face authentication apparatus control program; a computer-readable recording medium; and a method of controlling the face authentication apparatus.

### Solution to Problem

In order to solve the foregoing problems, a face authentication apparatus according to the present invention is a face authentication apparatus comprising extracting means that determines degrees of similarity between incoming input face image and a plurality of registered facial information items that have been preregistered, and then extracts the registered facial information item having the degree of similarity to the input face image of not less than a predetermined value, the registered facial information items being respectively registered together with attribute information items on persons related to the registered facial information items, the face authentication apparatus further comprising: identifying means that, if the extracting means extracts the plurality of registered facial information items, identifies which of the plurality of registered facial information items that have been extracted by the extracting means is the registered facial information item containing a person related to the input face image, the identifying means comprising: estimating means that analyzes the input face image, estimates an attribute of the person related to the input face image from a result of the analysis, and then outputs estimated attribute information item that represents the attribute thus estimated; and comparing means that makes comparison between the attribute information item having been estimated by the estimating means and the attribute information items on the persons related to the plurality of registered facial information items, whereby the registered facial information item containing the person related to the input face image is identified on a basis of a result of the comparison made by the comparing means.

In order to solve the foregoing problems, a method of controlling a face authentication apparatus according to the present invention is a method of controlling a face authentication apparatus that comprises extracting means that determines degrees of similarity between incoming input face image and a plurality of registered facial information items that have been preregistered, and then extracts the registered facial information item having the degree of similarity to the input face image of not less than a predetermined value, the registered facial information items being respectively registered together with attribute information items on persons related to the registered facial information items, the method comprising: an identifying step of identifying means included in the face authentication apparatus, if the extracting means extracts the plurality of registered facial information items, identifying which of the plurality of registered facial information items thus extracted is the registered facial information item containing a person related to the input face image, the identifying step comprising: an estimating step of analyzing the input face image, estimating an attribute of the person related to the input face image from a result of the analysis, and then outputting an estimated attribute information item that represents the attribute thus estimated; and a comparing step of making comparison between the attribute information item thus estimated in the estimating step and the attribute information items on the persons related to the plurality of registered facial information items, whereby the registered facial information item containing the person related to the input face image is identified on a basis of a result of the comparison made in the comparing step.

According to the above configuration, the face authentication apparatus determines the degrees of similarity between the incoming input face image and the registered facial information items that have been preregistered, and then extracts the registered facial information item(s) having the degree of similarity to the input face image of not less than the predetermined value.

Note that the input face image and the registered facial information item are images each containing a face of a person. Further, the degree of similarity is an index indicating the extent to which the input face image is similar to the registered facial information item. For example, as in a publicly known face authentication technique, the degree of similarity can be determined by extracting characteristics from face images and then evaluating the characteristics thus extracted. Besides, the registered facial information item is registered together with the attribute information item on the person related to the registered facial information item.

If the face authentication apparatus extracts the plurality of registered facial information items having the degree of similarity to the input face image of not less than the predetermined value, it identifies which of the plurality of registered facial information items is the registered facial information item containing the person related to the input face image.

Conventionally, registered facial information items each having the highest degree of similarity were outputted as a result of the authentication in most cases. However, there is a high possibility that the plurality of registered facial information items thus outputted represent persons who are very similar in appearance to each other. For example, the degrees of similarity between a target person and his/her parents, children, brothers, and sisters, and other close relatives obtained in the face authentication are close to one another in most cases. This is because the parents, children, brothers, and sisters, and other close relatives are similar to the target person in shape of a face. In addition, facial expressions, an angle of a face, intensity of illumination, and other conditions may have an effect on the calculation of the degrees of similarity and therefore cause errors of the degrees of similarity. Because of this, the degrees of similarity obtained in the face authentication procedure become close to one another sometimes.

In such a case, if the registered facial information items are extracted for the simple reason that they have the highest degree of similarity, another person who is very similar to the target person in appearance may be incorrectly outputted as a result of the authentication. That is why the determination based on an index other than the degree of similarity is desired in the face authentication procedure.

On the contrary, a face authentication apparatus according to the present invention identifies the registered facial information item in the following manner. That is, first of all, the face authentication apparatus analyzes the input face image, estimates an attribute of a person related to the input face image from the result of the analysis, and then outputs the estimated attribute information item representing information thus estimated. Thereafter, the face authentication apparatus makes comparison between the estimated attribute information item thus outputted and the attribute information items on persons related to the plurality of registered facial information items. Thus, the face authentication apparatus of the present invention performs authentication based on not only the degree of similarity but also the attribute information items as criteria of the determination.

Then, the face authentication apparatus according to the present invention identifies the registered facial information item containing the person related to the input face image on the basis of the result of the comparison between the attribute information items. This makes it possible to distinguish between, for example, persons who are similar in appearance as if they are family members, by their different attribute information items. Consequently, an accuracy of the face authentication procedure improves.

Still further, the above-described identifying process is performed in a case where the face authentication apparatus extracts the plurality of registered facial information items having the degree of similarity to the input face image of not less than the predetermined value. This eliminates the need for estimating the attribute every time the face authentication procedure is performed. On the contrary, the conventional art (e.g. Patent Literatures 4 and 5) is configured such that the attribute is estimated every time the face authentication procedure is performed. That is, the above-described configuration of the face authentication apparatus according to the present invention such that the attribute is estimated after the face authentication procedure is superior to the configuration of the conventional art in terms of efficiency of the estimation process.

This yields the effect of further improving an accuracy of the face authentication procedure without need for any special video equipment and others.

### Advantageous Effects of Invention

A face authentication apparatus according to the present invention is configured such that registered facial information items are respectively registered together with attribute information items on persons related to the registered facial information items, the face authentication apparatus comprises identifying means that, if extracting means extracts the plurality of registered facial information items, identifies which of the plurality of registered facial information items that have been extracted by the extracting means is the registered facial information item containing a person related to the input face image, the identifying means comprises: estimating means that analyzes the input face image, estimates an attribute of the person related to the input face image from a result of the analysis, and then outputs an estimated attribute information item that represents the attribute thus estimated; and comparing means that makes comparison between the attribute information item having been estimated by the estimating means and the attribute information items on the persons related to the plurality of registered facial information items, whereby the registered facial information item containing the person related to the input face image is identified on a basis of a result of the comparison made by the comparing means.

A method of controlling a face authentication apparatus according to the present invention is such that the registered facial information items are respectively registered together with attribute information items on persons related to the registered facial information items, the method comprises: an identifying step of identifying means included in the face authentication apparatus, if the extracting means extracts the plurality of registered facial information items, identifying which of the plurality of registered facial information items thus extracted is the registered facial information item containing a person related to the input face image, the identifying step comprises: an estimating step of analyzing the input face image, estimating an attribute of the person related to the input face image from a result of the analysis, and then outputting an estimated attribute information item that represents the attribute thus estimated; and a comparing step of making comparison between the attribute information item thus estimated in the estimating step and the attribute information items on the persons related to the plurality of registered facial information items, in the identifying step, identifying the registered facial information item containing the person related to the input face image on a basis of a result of the comparison made in the comparing step.

This yields the effect of further improving an accuracy of the face authentication procedure without need for any special video equipment and others.

Additional objects, features, and strengths of the present invention will be made clear by the description below. Further, the advantages of the present invention will be evident from the following explanation in reference to the drawings.

### Brief Description of Drawings

Fig. 1

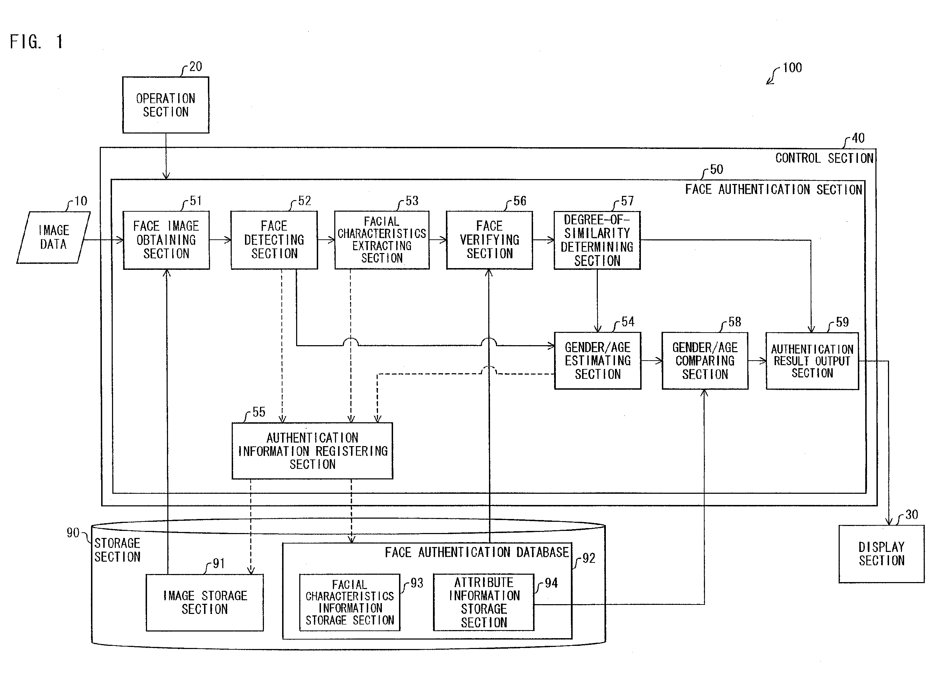

   Fig. 1 shows an embodiment of the present invention and is a block diagram schematically showing the configuration of a face authentication apparatus.
Fig. 2

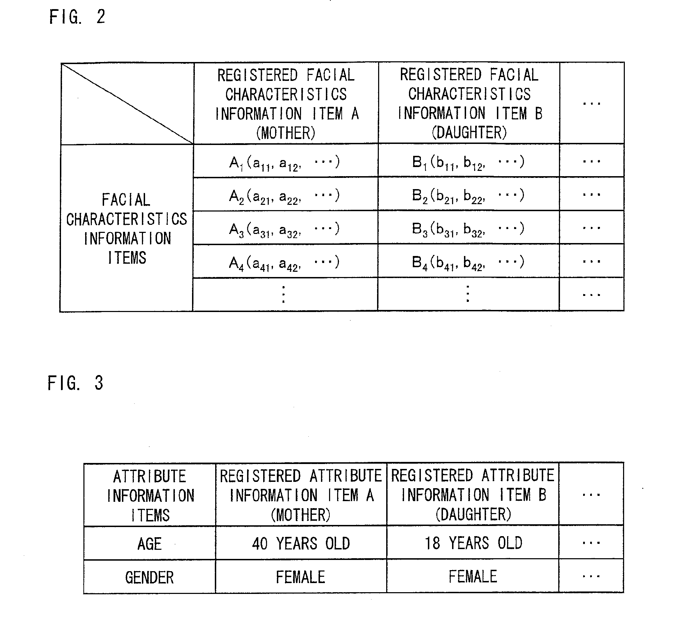

   Fig. 2 is a view showing an example of a registered facial characteristics information items stored in a facial characteristics information storage section.
Fig. 3
   Fig. 3 is a view showing an example of registered attribute information items stored in an attribute information storage section.
Fig. 4

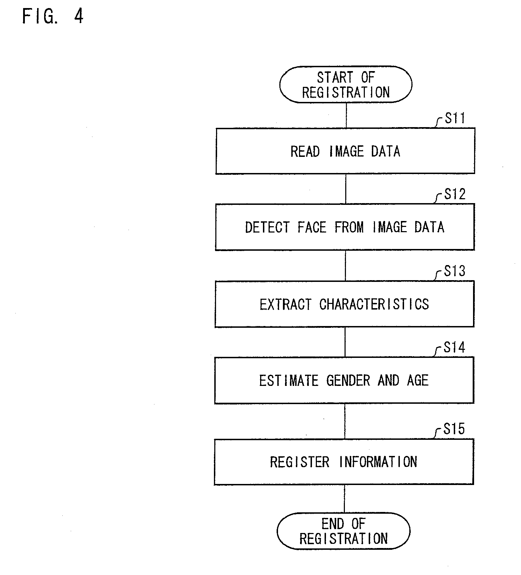

   Fig. 4 is a flowchart showing the flow of registration procedure performed by the face authentication apparatus.
Fig. 5

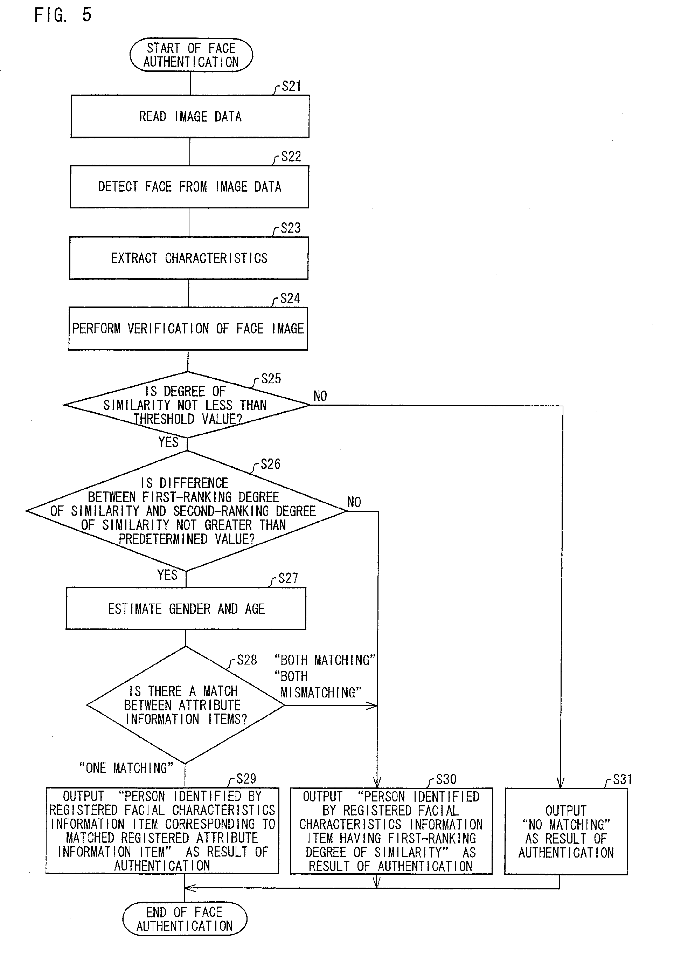

   Fig. 5 is a flowchart showing the flow of face authentication procedure performed by the face authentication apparatus.

### Description of Embodiments

The following will describe more details of the present invention according to embodiments of the present invention. However, these embodiments are not intended to limit the scope of the present invention.

The following will describe one embodiment of the present invention with reference to Figs. 1 through 5.

### (Configuration of a face authentication apparatus)

First of all, a schematic configuration of a face authentication apparatus (person image search system) 100 according to the present embodiment with reference to Fig. 1. Fig. 1 is a block diagram schematically showing the configuration of the face authentication apparatus 100. The face authentication apparatus 100 performs face authentication of input image data through preregistered face authentication information for identifying a person. The face authentication is a process for determining if a person represented by input image data matches a person identified by registered face authentication information.

As shown in Fig. 1, the face authentication apparatus 100 includes an operation section 20, a display section 30, a control section 40, and a storage section 90.

The operation section 20 accepts various entries from a user and is realized by: a pointing device including input-use buttons, a keyboard, a ten-key numeric pad, a mouse, and the like; a touch panel; or other input device. The operation section 20 generates operation data according to accepted user's operation and then transmits the operation data thus generated to the control section 40.

The display section 30 performs display of a screen for providing information to the user. The display section 30 displays various information items, such as characters and images, on a display screen on the basis of screen data having been received from the control section 40. The display section 30 is realized by a display device such as an LCD (Liquid Crystal Display), a PDP (Plasma Display Panel), or an EL (electroluminescence) display, for example.

The control section 40 performs centralized control of various functions of the face authentication apparatus 100. The control function of the control section 40 is realized by a processor, such as a CPU (Central Processing Unit), executing a control program. The control program may be stored in the storage section 90 that is a storage element such as RAM (Random Access Memory) or flash memory. Alternatively, the control program may be installed on a hard disk or the like and read from the hard disk or the like for use. Details of the control section 40 will be described later.

The storage section 90 stores various data items and programs. Examples of the storage section 90 include: a nonvolatile storage device such as hard disk; ROM (Read Only Memory) that is read-only semiconductor memory storing programs required for operations of the control section 40 and fixed data for use in various controls; RAM or the so-called working memory temporarily storing data for use in computations, results of the computations, and the like; and rewritable nonvolatile memory (e.g. flash memory) storing various data items such as setting data. Details of the storage section 90 will be described later.

### (Specific components in the control section and the storage section)

The following will describe essential components of the control section 40 and the storage section 90 with reference to Fig. 1 again. Supplemental remarks on Fig. 1 are as follows. As described previously, the face authentication apparatus 100 performs face authentication of input image data through preregistered face authentication information. That is, the procedures performed by the face authentication apparatus 100 fall into two broad categories: registration procedure; and face authentication procedure. In Fig. 1, connections between the components (data flow from one component to another) of the face authentication apparatus 100 in the registration procedure only are indicated by dotted line arrows. Further, connections between the components (data flow from one component to another) of the face authentication apparatus 100 in the face authentication procedure only or both in the face authentication procedure and in the registration procedure are indicated by solid line arrows.

### (Storage section)

First, the storage section 90 will be described in detail. As shown in Fig. 1, the storage section 90 includes an image storage section 91 and a face authentication database 92.

The image storage section 91 stores image data to be used in face authentication by the face authentication section (identifying means) 50. In the present embodiment, the image data is assumed to be data of an image including at least a face region of a person. One or more persons may be represented by the image data.

The image data can be supplied to the face authentication apparatus 100 and stored in the image storage section 91, for example, in any of the following manners. The face authentication apparatus 100 may be provided with an image capturing section (not shown) for capturing an image of a subject, so that image data 10 having been obtained by image capturing by the image capturing section is stored in the image storage section 91. The image capturing section may be realized by a CCD (Charge Coupled Device), a CMOS (Complementary Metal Oxide Semiconductor), or the like photoelectric conversion element.

Alternatively, the face authentication apparatus 100 may be provided with a communication section (not shown) for performing communications with an external entity so that the face authentication apparatus 100 is accessible to a network. In this case, the face authentication apparatus 100 obtains the image data 10 from the external entity via the network, and the obtained image data 10 is stored in the image storage section 91.

The face authentication database 92 is a database into which (i) the image data to be used in the face authentication procedure by the face authentication section 50 and (ii) data having been extracted from the image data are stored. The face authentication database 92 is constituted by a facial characteristics information storage section 93 and an attribute information storage section 94.

The following will describe the facial characteristics information storage section 93 and the attribute information storage section 94 with reference to Figs. 2 and 3. Fig. 2 is a view showing an example of registered facial characteristics information items (registered facial information items) stored in the facial characteristics information storage section 93. Fig. 3 is a view showing an example of registered attribute information items stored in the attribute information storage section 94.

The facial characteristics information storage section 93 is a database for storing the facial characteristics information items to be used in a face verification process by a face verifying section 56. The facial characteristics extracting section 53 outputs the facial characteristics information items on the basis of input facial image data, and an authentication information registering section 55 then registers the facial characteristics information items into the facial characteristics information storage section 93. The facial characteristics information items may contain the input face image data that is a source of the facial characteristics information items.

The registration procedure is a procedure for registering the facial characteristics information items having been obtained from the input face image data into the face authentication database 92. In the face authentication apparatus 100 according to the present embodiment, the facial characteristics information items, which are characteristics extracted from a facial image that is contained in the input face image data (image data) to be supplied, can be preregistered into the face authentication database 92.

With reference to Fig. 2, the following will describe the example of the facial characteristics information items registered in the facial characteristics information storage section 93.

As shown in Fig. 2, the facial characteristics information storage section 93 stores the registered facial characteristics information items by which particular persons A and B are identified through the face authentication. In the example shown in Fig. 2, the person A (mother) and the person B (daughter) are in a mother-daughter relationship. Further, each of the registered facial characteristics information items has identification information attached thereto. By the identification information, the registered facial characteristics information item can be uniquely identified. For ease of explanation, in the present embodiment, it is assumed that identification information of the person A (mother) is identification information A, and identification information of the person B (daughter) is identification information B. That is, a registered facial characteristics information item A is data by which the mother is to be identified through the face authentication, a registered facial characteristics information item B is data by which the daughter is to be identified through the face authentication.

Each of the registered facial characteristics information items contains a plurality of facial characteristics information items, which are information of facial characteristics of that person. In the example shown in Fig. 2, the registered facial characteristics information item A contains facial characteristics information items A₁ through A₄ and more, which are facial characteristics information items for identifying the mother. These facial characteristics information items A₁ through A₄ correspond to facial characteristics information items extracted by the facial characteristics extracting section 53 from one image data item. Note that the facial characteristics information items A₁ through A₄ may contain the input face image data itself.

As shown in the example of Fig. 2, the registered facial characteristics information item A is made up of a plurality of facial characteristics information items, for the purpose of increasing an accuracy of verification performed by the face verifying section 56. However, the present embodiment is not limited to the above example. It is essential only that the registered facial characteristics information item A contains at least one facial characteristics information item. Further, the facial characteristics information item A₁ contains information items a₁₁, a₁₂, ... regarding a plurality of characteristics having been extracted by the facial characteristics extracting section 53. The same goes for the facial characteristics information items A₂ through A₄ and more. Details of the information items a₁₁, a₁₂, ... regarding the characteristics will be described later.

The registered facial characteristics information item B has a data structure similar to that of the registered facial characteristics information item A, and an explanation thereof is therefore omitted.

The attribute information storage section 94 is a database for storing attribute information items through which a gender/age comparing section 58 performs a gender and generation comparison process. The gender/age estimating section 54 outputs the attribute information item on the basis of the input face image data, and the authentication information registering section 55 registers the attribute information item into the attribute information storage section 94. With reference to Fig. 3, the following will describe an example of the attribute information items stored in the attribute information storage section 94.

As shown in Fig. 3, the attribute information storage section 94 stores respective attribute information items of the person A (mother) and the person B (daughter). The facial characteristics information storage section 93 and the attribute information storage section 94 use common identification information items. In the example shown in Fig. 3, the registered attribute information item A and the registered attribute information item B respectively correspond to the registered facial characteristics information item A and the registered facial characteristics information item B, both of which are stored in the facial characteristics information storage section 93.

In the example shown in Fig. 3, the registered attribute information item is made up of an age (generation) and a gender.

In the registered attribute information item A shown in Fig. 3, "40 years old" is set for age, and "female" is set for gender.

Meanwhile, in the registered attribute information item B, "18 years old" is set for age, and "female" is set for gender.

Note that the registered facial characteristics information item stored in the facial characteristics information storage section 93 and the registered attribute information item stored in the attribute information storage section 94 may be manually entered and set by the user. For example, the information entered by the user via the operation section 20 may be obtained by the face authentication section 50 and then registered into the face authentication database 92. Further, the information to be registered in the face authentication database 92 may be supplied from an entity outside the face authentication apparatus 100. For example, the information can be supplied from an entity outside the face authentication apparatus 100 through the use of a removable storage medium, or the information can be obtained from an entity outside the face authentication apparatus 100 through communications via a network.

### (Control section)

Next, the following will describe the control section 40 in detail. As shown in Fig. 1, the control section 40 includes the face authentication section 50 for performing the face authentication procedure. The face authentication section 50 performs the face authentication procedure in accordance with the operation data transmitted from the operation section 20. More specifically, the face authentication section 50 includes: a face image obtaining section (extracting means) 51; a face detecting section (extracting means) 52; the facial characteristics extracting section (extracting means) 53; the gender/age estimating section (estimating means) 54; the authentication information registering section 55; the face verifying section (extracting means) 56; a degree-of-similarity determining section (estimating means) 57; the gender/age comparing section (comparing means) 58; and an authentication result output section (identifying means) 59.

The face image obtaining section 51 obtains the image data to be used in the registration procedure and the face authentication procedure. In the registration procedure and the face authentication procedure, the face image obtaining section 51 reads the image data from the image storage section 91 and then transfers the read image data to the face detecting section 52.

Note that in the registration procedure, the face image obtaining section 51 may obtain the image data 10 supplied from the external entity and then transfer the obtained image data 10 to the face detecting section 52, as described previously. In this case, the face image obtaining section 51 may store the image data 10 supplied from the external entity in the image storage section 91.

The face detecting section 52 detects a region corresponding to a person's face from the image data transferred from the face image obtaining section 51, and then outputs the input face image data, which is data of an image of the detected face. The face detecting section 52 may detect a plurality of faces from the image data, and the face detecting section 52 may be configured to output a face count, a location of a face in the image data, etc. Further, the face detecting section 52 may determine that an image of a person is not represented by the image data if no person's face is detected from the image data. This can occur, for example, in a case where the image data represents an image of a scenic shot. In this case, the face authentication section 50 may abort the face authentication procedure.

Note that the technique disclosed in Patent Literature 1 can be used for the face detection process performed by the face detecting section 52. However, this is not intended to limit the face detection process performed by the face detecting section 52. Alternatively, a well-known face detection technique can be used.

The facial characteristics extracting section 53 extracts, from the input face image data outputted from the face detecting section 52, characteristics contained in the input face image data, and then outputs information items regarding the characteristics thus extracted, as the facial characteristics information items. Examples of the characteristics extracted by the facial characteristics extracting section 53 include parts recognized as eyes, a nose, and a mouth in the person's face contained in the input face image data. Further, examples of the information items regarding the characteristics include locations and sizes of the parts.

Now, the following will more specifically describe the facial characteristics information items generated by the facial characteristics extracting section 53 with reference to Fig. 2 again. The registered facial characteristics information item A is taken as an example. The facial characteristics information item A₁ may be generated from one input face image data item by the facial characteristics extracting section 53. In this case, the information items regarding the characteristics such as eyes and a nose correspond to a₁₁ and a₁₂.

Note that a well-known technique can be used for extraction of the characteristics and generation of the facial characteristics information items, both of which are performed by the facial characteristics extracting section 53.

The gender/age estimating section 54 analyzes the input face image data having been outputted from the face detecting section 52, estimates a gender and an age of the person represented by the input face image data, and then outputs the estimation result as the attribute information. The gender/age estimating section 54 may estimate a gender and an age by using the technique disclosed in Non-Patent Literature 1. However, this is not the only possibility. Alternatively, for the gender and age estimation process performed by the gender/age estimating section 54, a well-known technique may be employed appropriately. Further, the gender/age estimating section 54 may make estimation of generations that fall into, for example, the following categories: 10's, 20's, ... or child, juvenile, young adult, elderly, etc.

The gender/age estimating section 54 performs the estimation process in both the registration procedure and the face authentication procedure. The gender/age estimating section 54 outputs the attribute information items to the authentication information registering section 55 in the registration procedure, but to the gender/age comparing section 58 in the face authentication procedure.

The authentication information registering section 55 is a section for registering information to be used in the face authentication procedure into the face authentication database 92 in the registration procedure. The information to be used in the face authentication procedure includes the facial characteristics information items outputted from the facial characteristics extracting section 53 and the attribute information items outputted from the gender/age estimating section 54. Note that the authentication information registering section 55 may be configured to register the input face image data having been outputted from the face detecting section 52 into the face authentication database 92.

The face verifying section 56 sequentially reads the registered facial characteristics information items from the facial characteristics information storage section 93, and then verifies the input face image data outputted from the face detecting section 52 on the basis of the registered facial characteristics information items thus read. The verification process performed by the face verifying section 56 is performed on the basis of one or more facial characteristics information items contained in the registered facial characteristics information item. Besides, the face verifying section 56 is allowed to use the registered attribute information items in the verification process.

Furthermore, in the present embodiment, the degree of similarity between persons identified by the respective registered facial characteristics information items and the person represented by the input face image data is calculated in the verification process performed by the face verifying section 56. The degree of similarity can be expressed by a numeric value. The face verifying section 56 determines if the calculated degree of similarity is not less than a predetermined threshold value.

If the face verifying section 56 determines that there have been no registered facial characteristics information items having the degree of similarity that is not less than the predetermined threshold value, the face verifying section 56 outputs "no matching" as the result of the verification.

On the other hand, if the face verifying section 56 determines that there have been one or more registered facial characteristics information items whose degree of similarity is not less than the predetermined threshold value, the face verifying section 56 outputs such registered facial characteristics information item(s) as the result of the verification.

Assume that there has been one registered facial characteristics information item contained in the verification result provided by the face verifying section 56. This indicates that the person represented by the input face image data has been determined as matching the person identified by that registered facial characteristics information item.

For the verification process performed by the face verifying section 56, a well-known technique disclosed in Patent Literature 2 can be employed, for example. In addition, the configuration of the face verifying section 56 is not limited to the above-described configuration. The design of the face verifying section 56 may be changed appropriately.

If the verification result having been outputted from the face verifying section 56 includes a plurality of registered facial characteristics information items, the degree-of-similarity determining section 57 determines if the registered facial characteristics information items fall within a similarity range.

More specifically, the degree-of-similarity determining section 57 calculates a difference between the highest degree of similarity and the second highest degree of similarity, among a plurality of degrees of similarity having been calculated by the face verifying section 56. Then, the degree-of-similarity determining section 57 determines if the difference thus calculated is not greater than a predetermined value of a similarity range limit.

There is a high possibility that the registered facial characteristics information item having the highest degree of similarity and the registered facial characteristics information item having the second highest degree of similarity indicate persons who are very similar in appearance. A small difference in degree of similarity means that both of the registered facial characteristics information items indicate persons who are particularly similar in appearance, and it is highly likely that authentication is erroneously made in the authentication procedure. In view of this, the degree-of-similarity determining section 57 determines if the registered facial characteristics information item having the highest degree of similarity and the registered facial characteristics information item having the second highest degree of similarity indicate persons who are particularly similar in appearance, by using the predetermined value of similarity range limit.

That is, if the calculated difference is not greater than the predetermined value of similarity range limit, the individual persons identified by the two registered facial characteristics information items can be particularly similar in appearance. Therefore, the determination only by the degree of similarity in the face authentication procedure may cause an erroneous result.

If the calculated difference is not greater than the predetermined value of similarity range limit, the degree-of-similarity determining section 57 instructs the gender/age estimating section 54 to perform gender and age estimating process with respect to the input image data, and outputs, as a result of the determination, "not greater than the predetermined value of similarity range limit".

On the other hand, if the calculated difference is greater than the predetermined value of similarity range limit, the degree-of-similarity determining section 57 outputs, as a result of the determination, "greater than the predetermined value of similarity range limit".

Note that the predetermined value of similarity range limit is any value that can be defined as appropriate by a user. Further, a default value may be preset for the predetermined value of similarity range limit. For example, the default value can be "a value equivalent to a one-tenth of the highest degree of similarity".

In the face authentication procedure, the gender/age comparing section 58 determines if the attribution information item of the input face image data matches the registered attribute information item stored in the attribute information storage section 94, and then outputs a result of the determination.

More specifically, the gender/age comparing section 58 determines the degree of matching between both of the attribution information items. First, if the determination result having been outputted from the degree-of-similarity determining section 57 is "not greater than the predetermined value of similarity range limit", the gender/age comparing section 58 obtains the attribute information item of the input face image data from the gender/age estimating section 54.

Further, the gender/age comparing section 58 reads, from the attribute information storage section 94, the respective attribute information items corresponding to the registered facial characteristics information item having the highest degree of similarity and the registered facial characteristics information item having the second highest degree of similarity.

Then, the gender/age comparing section 58 compares the attribute information items having been read from the attribute information storage section 94 with the attribute information items of the input face image data.

In the present embodiment, the gender/age comparing section 58 performs comparisons of "age" and "gender" contained in the attribute information items, as comparison of the attribute information items.

Now, the comparison process performed by the gender/age comparing section 58 will be described.

The gender/age comparing section 58 performs comparison of the "age" by determining if there is a match in age, and determines if an absolute value of a difference between the age contained in the registered attribute information item and the age contained in the attribute information item of the input face image data falls within a predetermined range. If the absolute value falls within the predetermined range, the gender/age comparing section 58 determines the persons indicated by the registered attribute information item and the attribute information item as "being of the same generation". The following descriptions assume that the determination result that "the persons are of the same generation" by the gender/age comparing section 58 includes a determination result that "the persons are of the same age". However, this is not the only possibility. Alternatively, the determination result obtained by the gender/age comparing section 58 may be "the persons are of the same age" only.

The predetermined range can be preset by the user. Alternatively, the gender/age comparing section 58 may be configured to determine that "the persons are of the same generation" if the absolute value of the difference in age is not greater than 10, as a default of the predetermined range.

The gender/age comparing section 58 performs comparison of the "gender" by determining if there is a match in gender.

If the result of the "age" comparison between the attribute information items shows that "the persons are of the same generation", and there is a match in "gender", the gender/age comparing section 58 determines that there is a match between the attribute information items.

The following will describe what the gender/age comparing section 58 determines on the basis of the result of the comparison process. In the present embodiment, if the attribute information items of the input face image data matches either of (i) the registered attribute information item corresponding to the registered facial characteristics information item having the highest degree of similarity and (ii) the registered attribute information item corresponding to the registered facial characteristics information item having the second highest degree of similarity, the gender/age comparing section 58 outputs, as the result of the determination, a person identified by the facial characteristics information item corresponding to the registered attribute information item (i) or (ii) which matches the attribute information item of the input face image data.

The authentication result output section 59 compiles the result of the verification made by the face verifying section 56, the result of the determination made by the degree-of-similarity determining section 57, and the result of the comparison made by the gender/age comparing section 58, to then output a result of the authentication. The authentication result output section 59 causes the display section 30 to show the authentication result having been outputted from the authentication result output section 59.

### (Flows of the procedures)

Next, the following will describe flows of the respective procedures in the face authentication apparatus 100 with reference to Figs. 4 and 5. Fig. 4 is a flowchart showing a flow of the registration procedure in the face authentication apparatus 100. Further, Fig. 5 is a flowchart showing a flow of the face authentication procedure in the face authentication apparatus 100.

### (Flow of the registration procedure)

First, the following will describe the flow of the registration procedure with reference to Fig. 4. In response to the user's operation for providing an instruction to perform the registration procedure through the operation section 20, the face authentication section 50 starts the registration procedure. In the operation for providing the instruction to perform the registration procedure, image data targeted for the registration procedure is specified.

When the registration procedure is started, the face image obtaining section 51 reads the image data from the image storage section 91 and then transfers the thus read image data to the face detecting section 52 (S11). Next, the face detecting section 52 detects a person's face from the image data and then outputs the input face image data (S12). Subsequently, the facial characteristics extracting section 53 extracts the facial characteristics contained in the input face image data from the input face image data having been outputted from the face detecting section 52 and then outputs the facial characteristics information (S13). The gender/age estimating section 54 analyzes the input face image data to estimate gender and age of the person represented by the input face image data, and then outputs a result of the estimation as attribute information items (S14).

Finally, the authentication information registering section 55 registers information items having been outputted from the facial characteristics extracting section 53 and the gender/age estimating section 54 to the face authentication database (S15). Specifically, the authentication information registering section 55 registers facial characteristics information having been outputted from the facial characteristics extracting section 53 to the facial characteristics information storage section 93, and registers attribute information having been outputted from the gender/age estimating section 54 to the attribute information storage section 94. Note that the authentication information registering section 55 may store input image data having been detected by the face detecting section 52 in the image storage section 91. The face authentication apparatus 100 enables a plurality of persons targeted for the face authentication to be registered into the face authentication database 92 by repeating the above-described procedure. Further, in the face authentication apparatus 100, the input face image data can be registered into the face authentication database 92.

According to the above-described flow, the registration procedure is performed.

### (Flow of the face authentication procedure)

Next, the following will describe the flow of the face authentication procedure with reference to Fig. 5. In response to the user's operation for providing an instruction to perform the face authentication procedure through the operation section 20, the face authentication section 50 starts the face authentication procedure. In the operation for providing the instruction to perform the face authentication procedure, image data targeted for the face authentication procedure is specified.

The processes in the steps S21 through S23 are the same as those in the steps S11 through 13, and explanation thereof is therefore omitted.

When the facial characteristics extracting section 53 outputs the facial characteristics information of the input image data, the face verifying section 56 performs the verification process (S24). Specifically, the face verifying section 56 sequentially read the registered facial characteristics information items having been stored in the facial characteristics information storage section 93, and then performs the verification process using (i) the facial characteristics information items contained in the registered facial characteristics information items thus read and (ii) characteristics information items of the input image data. As a result of the verification process, the face verifying section 56 finds the degree of similarity between a person identified by each of the registered facial characteristics information items and a person represented by the input image data. Subsequently, the face verifying section 56 determines if the degree of similarity having been found for each of the registered facial characteristics information items is not less than the threshold value (S25).

If the result of the determination performed by the face verifying section 56 shows that there have been no registered facial characteristics information items having degree of similarity that is not less than a predetermined threshold value (NO in S25), the authentication result output section 59 outputs "no matching" as a result of the authentication to the display section 30 (S31). Further, if the result of the determination performed by the face verifying section 56 shows that there have been one registered facial characteristics information item having the degree of similarity that is not less than the predetermined threshold value, the authentication result output section 59 outputs "person identified by the registered facial characteristics information item having the first-ranking degree of similarity" as a result of the authentication to the display section 30. Thereafter, in either case, the procedure is completed.

On the other hand, if the result of the determination performed by the face verifying section 56 shows that there have been one or more registered facial characteristics information items having the degree of similarity of not less than the predetermined threshold value (YES in S25), the degree-of-similarity determining section 57 calculates a difference between the first-ranking degree of similarity and the second-ranking degree of similarity, and then determines if the difference thus calculated is not greater than the predetermined value of similarity range limit (S26).

If the result of the determination performed by the degree-of-similarity determining section 57 shows that the difference thus calculated is greater than the predetermined value of similarity range limit (NO in S26), the authentication result output section 59 outputs "person identified by the registered facial characteristics information item having the first-ranking degree of similarity" as the result of the authentication (S30).

If the result of the determination performed by the degree-of-similarity determining section 57 shows that the thus calculated difference is not greater than the predetermined value of similarity range limit (NO in S26), the gender/age estimating section 54 analyzes the input face image data having been outputted from the face detecting section 52 to estimate gender and age (generation) of the person represented by the input face image data, and then outputs the result of the estimation as the attribute information items (S27).

Subsequently, the gender/age comparing section 58 compares the registered attribute information items corresponding to the registered facial characteristics information items having the first-ranking and second-ranking degrees of similarity with the attribute information items that have been estimated from the input face image data by the gender/age estimating section 54 to determine if there is a match. That is, the gender/age comparing section 58 determines if there is a match between the gender having been estimated from the input face image data by the gender/age estimating section 54 and the gender registered in the registered attribute information item and determines if a difference between the age having been estimated from the input face image data by the gender/age estimating section 54 and the age registered in the registered attribute information item falls within a predetermined range (S28).

If the result of the determination performed by the gender/age comparing section 58 shows that there is a match between the attribute information item having been estimated from the input face image data and both of the registered attribute information items corresponding to the registered facial characteristics information items having the first-ranking and second-ranking degrees of similarity, and if the result of the determination shows that there is no match ("both matching" and "both mismatching" in S28), the authentication result output section 59 outputs "person identified by the registered facial characteristics information item having the first-ranking degree of similarity" as the result of the authentication (S30). Thereafter, the procedure is completed.

On the other hand, if the result of the determination shows that the attribute information item having been estimated from the input face image data matches either of the registered attribute information items of the registered facial characteristics information items having the first-ranking and second-ranking degrees of similarity ("one matching" in S28), the authentication result output section 59 outputs "person identified by the registered facial characteristics information item corresponding to the matched registered attribute information item" as the result of the authentication (S29). Thereafter, the procedure is completed.

In a case where the image data targeted for the face authentication procedure contains a plurality of person's faces and a plurality of input face image data items are therefore created, the processes in the steps 23 through S31 above should be repeated for each of the input face image data items.

Further, in a case where a plurality of image data items targeted for the face authentication procedure are selected, the processes in the steps S21 through S31 should be repeated for each of the image data items. In this case, the face authentication apparatus 100 may be configured such that the face authentication section 50 accepts the selection of the registered facial characteristics information item for identification of a person targeted for output as a result of the authentication, and the authentication result output section 59 outputs, as the result of the authentication, image data item representing a person that matches the person identified by the registered facial characteristics information. According to this configuration, it is possible to search for image data representing a specific person through image data items stored in the image storage section 91. In other words, with this configuration, it is possible to construct the so-called person image search system.

According to the above-described flow, the face authentication procedure is performed.

With reference to Figs. 2 and 3 again, the following will more specifically describe the flow of the face authentication procedure in the present embodiment. The following will describe a case where the input face image data is data of an image of a daughter in the examples shown in Figs. 2 and 3. Since a mother and a daughter are generally similar to each other in shape of a face, appearance of a face, and other facial features, the degree of similarity of the registered facial characteristics information item A and the degree of similarity of the registered facial characteristics information item B are both expected to be high. Besides, it can be considered that facial expressions, an angle of a face, intensity of illumination, and other conditions may have an effect on the calculation of the degrees of similarity.

In such a case, it is determined in S25 that the degree of similarity is greater than the threshold value (YES in S25), and a difference in the degree of similarity between the registered facial characteristics information items A and B is expected to be not greater than the predetermined value of similarity range limit (YES in S26).

In S27, from the input face image data which is the image data of the daughter, gender and age of the daughter are estimated to be "female" and "18", respectively.

Therefore, as a result of the comparison between the attribute information items, the attribute information items having been estimated from the input face image data matches the registered attribute information item B, but does not match the registered attribute information item A.

Consequently, even if the result of the calculation shows that the degree of similarity of the registered facial characteristics information item B is lower than the degree of similarity of the registered facial characteristics information item A with the assumption that the previously-described conditions affect the calculation of the degrees of similarity, the authentication result output section 59 outputs "daughter" as the result of the authentication since the attribute information items match the registered attribute information items.

Thus, according to the face authentication apparatus 100 according to the present embodiment, it is possible to prevent the occurrence of incorrect recognition by using the attribute information items.

### (Operation and Effect)

As described above, a face authentication apparatus 100 includes the face verifying section 56 that determines degrees of similarity between incoming input face image data and a plurality of registered facial characteristics information items that have been preregistered, and then extracts the registered facial characteristics information item having the degree of similarity to the input face image data of not less than a predetermined value, the registered facial characteristics information items being respectively registered together with registered attribute information items on persons related to the registered facial characteristics information items, the face authentication apparatus 100 further including: the face authentication section 50 that, if the face verifying section 56 extracts the plurality of registered facial characteristics information items, identifies which of the plurality of registered facial characteristics information items that have been extracted by the face verifying section 56 is the registered facial characteristics information item containing a person related to the input face image data, the face authentication section 50 including: the gender/age estimating section 54 that estimates an attribute information item on the person related to the input face image data and then outputs the attribute information item thus estimated; and gender/age comparing section 58 that makes comparison between the attribute information item having been outputted from the gender/age estimating section 54 and the registered attribute information items on the persons related to the plurality of registered facial characteristics information items, whereby the registered facial characteristics information item is identified on the basis of a result of the comparison.

This yields the effect of improving an accuracy of the face authentication procedure without need for any special video equipment and others.

### (Modifications)

The following will describe preferable modifications of the present embodiment.

In the present embodiment, the degree-of-similarity determining section 57 calculates a difference between the highest degree of similarity and the second highest degree of similarity among the plurality of degrees of similarity having been calculated by the face verifying section 56 and then determines if the difference thus calculated is not greater than the predetermined value of similarity range limit. However, the degree-of-similarity determining section 57 may also include, as a target for the determination, the third or subsequent highest degrees of similarity. That is, the degree-of-similarity determining section 57 may also include, as a target for the determination, a degree of similarity lower than the highest degree of similarity by an amount which is not greater than the predetermined value of similarity range limit.

In this case, the increase of the number of targets for the determination may increase a load on the face authentication procedure. However, in a case where a difference between the highest degree of similarity and the third highest degree of similarity is not greater than the predetermined value of similarity range limit, a person identified by the registered facial characteristics information item having the third highest degree of similarity is expected to be similar in appearance to the person identified by the registered facial characteristics information item having the highest degree of similarity. Therefore, an accuracy of the face authentication procedure can be expected to improve.

In this case, settings such as the value of similarity range limit should be changed as appropriate in consideration of the trade-off between the accuracy of the face authentication procedure and the load on the face authentication procedure.

Further, the degree-of-similarity determining section 57 may notify the gender/age comparing section 58 of the registered facial characteristics information items having first to predetermined n-th highest degrees of similarity, so that the gender/age comparing section 58 performs comparison of the attribute information items between the thus notified registered facial characteristics information items having the first to predetermined n-th highest degrees of similarity. For example, this makes it possible that the top three degrees of similarity are targeted for the determination and comparison. Further, how many degrees of similarity in descending ranking of the degrees of similarity are to be targeted for the determination and comparison can be determined as appropriate by the user.

Still further, the gender/age comparing section 58 may be configured to determine the attribute information items as being "partially matching" if there is a match in "age" or "gender". Then, in S28, if there are both a "partially matching" attribute information item and a "mismatching" attribute information item, the authentication result output section 59 may output "person identified by the registered facial characteristics information item corresponding to the partially matching attribute information item" as a result of the determination.

In the present embodiment, the gender/age estimating section 54 estimates "age" and "gender" from the input image data. Optionally, the gender/age estimating section 54 may estimate "race". That is, the attribute information items may include an information item on "race" as well as the information items on "age" and "gender". In this case, the gender/age comparing section 58 should be configured to make comparison of "race". This enables determination of "race" as well in a case where persons of different races are registered in the face authentication database 92, and, in turn, enables improvement of an accuracy of the face authentication procedure. Note that a "race" estimation process performed by the gender/age estimating section 54 can be realized by using a publicly-known technique.

### (Other variations)

By employing the configuration in which the image-capturing section is provided to the present invention, it is possible to realize a digital camera capable of searching for photograph image data having been obtained by photographing persons through the face authentication procedure. Such a digital camera is beneficial because it enables easy search for photograph image data of a target person in these days of an enormous number of photograph image data items that can be stored in digital cameras in accordance with the trend toward increase in capacity of storage media.

Further, in a case where the present invention is arranged so as to be connected to a surveillance camera that capture images for a given time period or in real time and to receive image data from the surveillance camera, it is possible to construct a face authentication surveillance system that enables a person caught on the surveillance camera to be identified through the face authentication procedure. With such a face authentication surveillance system, it is possible to determine with a high degree of accuracy if a person caught on the surveillance camera is identical to a person having been registered, and the face authentication surveillance system is therefore used as an access control system, for example.

The present invention is not limited to the aforementioned embodiments and is susceptible of various changes within the scope of the accompanying claims. That is, an embodiment obtained by suitable combinations of technical means changed as appropriate within the scope of the accompanying claims is also included within the technical scope of the present invention.

Further, the present invention can be expressed as follows. As described above, a face authentication apparatus according to the present invention is preferably such that the estimating means outputs the estimated attribute information item for each registered facial information item having a specific degree of similarity that is lower than the highest degree of similarity by an amount falling within a predetermined range, among the thus determined degrees of similarity of the registered facial information items having been extracted by the extracting means.

Among the thus determined degrees of similarity of the registered facial information items having been extracted by the extracting means, each registered facial information item having a specific degree of similarity that is lower than the highest degree of similarity by an amount falling within the predetermined range, is highly likely to represent a person similar in appearance to the person identified by the registered facial information item having the highest degree of similarity, as compared to the registered facial information item having a degree of similarity that is lower than the highest degree of similarity by an amount falling outside the predetermined range. Therefore, it is also highly likely that incorrect recognition of the persons represented by these registered facial information items would occur in the face authentication.

In the situations that require the face authentication procedure to be performed with a high degree of efficiency, the comparison between attribute information items may be performed under significantly heavy load conditions. However, according to the above configuration, the estimated attribute information item is outputted only for the registered facial information item by which the incorrect recognition will be most probably caused in the face authentication. This eliminates output of the estimated attribute information item every time verification of the input face image is performed. Therefore, the above configuration is more advantageous than a configuration where the estimated attribute information item is outputted every time, in terms of processing efficiency.

Consequently, the effect of improving an accuracy of the face authentication procedure can be produced without significant loss in efficiency of the face authentication procedure.

A face authentication apparatus according to the present invention is preferably such that the estimating means outputs (i) the estimated attribute information item for a registered facial information item having the highest degree of similarity and (ii) the estimated attribute information item for a registered facial information item having a predetermined n-th highest degree of similarity, among the thus determined degrees of similarity of the registered facial information items having been extracted by the extracting means.

Among the thus determined degrees of similarity of the registered facial information items having been extracted by the extracting means, the registered facial information item having the highest degree of similarity and the registered facial information item having a predetermined n-th highest degree of similarity are highly likely to represent persons similar in appearance to each other, as compared to the other registered facial information items. Therefore, it is also highly likely that incorrect recognition of the persons represented by these registered facial information items would occur in the face authentication.

In the situations that require the face authentication procedure to be performed with a high degree of efficiency, the comparison between attribute information items may be performed under significantly heavy load conditions. However, according to the above configuration, the estimated attribute information item is outputted only for the registered facial information item by which the incorrect recognition will be most probably caused in the face authentication. This eliminates output of the estimated attribute information item every time verification of the input face image is performed. Therefore, the above configuration is more advantageous than a configuration where the attribute information item is estimated every time, in terms of processing efficiency.

Consequently, the effect of improving an accuracy of the face authentication procedure can be produced without significant loss in efficiency of the face authentication procedure.

A face authentication apparatus according to the present invention is preferably such that each of the attribute information items includes at least one of information items on age, gender, and race.

Each of the attribute information items includes at least one of information items on age, gender, and race. That is, it is possible to prevent the occurrence of incorrect recognition in the face authentication by using the information items on age, gender, and race.

A person image search system may be configured to include: a face authentication apparatus according to the present invention; a person image storage section for storing person images each of which represents at least a face region of a person; a registered facial information storage section for storing a plurality of registered facial information items; selecting means for selecting the registered facial information item from among the plurality of registered facial information items stored in the registered facial information storage section; image supplying means for reading the person image from the person image storage section, detecting the face region from the person image thus read, and then supplying the face region thus detected as an input face image to the face authentication apparatus; authentication result obtaining means for obtaining a result of face authentication made for the input face image having been supplied from the image supplying means from the face authentication apparatus; and searching means for searching, on a basis of the result having been obtained by the authentication result obtaining means, a person image containing a person represented by the registered facial information item having been selected by the selecting means through the person images stored in the person image storage section.

According to the above configuration, the person image representing at least a face region of a person is read, the face region of the person is detected from the person image thus read, and the face region thus detected is supplied as an input image to the face authentication apparatus. Thereafter, the result of the face authentication is obtained from the face authentication apparatus. Further, using the face authentication result thus obtained, the person image containing the person represented by the selected registered facial information item is searched for.

Thus, by incorporating the face authentication apparatus into the person image search system, it is possible to realize the person image search system that searches for a person image representing a target person with a high degree of accuracy.

Note that the face authentication apparatus may be realized by a computer. In this case, the present invention encompasses (i) a face authentication apparatus control program that causes the computer to realize the face authentication apparatus by causing the computer to operate each of the foregoing means and (ii) a computer-readable recording medium storing the face authentication apparatus program.

Finally, the blocks of the face authentication apparatus 100, particularly the following blocks provided in the face authentication section 50, i.e. the face image obtaining section 51, the face detecting section 52, the facial characteristics extracting section 53, the gender/age estimating section 54, the authentication information registering section 55, the face verifying section 56, the degree-of-similarity determining section 57, the gender/age comparing section 58, and the authentication result output section 59 may be constituted by hardware logic or realized by software by means of a CPU (central processing unit) as shown below.

That is, the face authentication apparatus 100 includes a CPU that executes the order of a control program for realizing the aforesaid functions, ROM (read only memory) that stores the control program, RAM (random access memory) that develops the control program in executable form, and a storage device (storage medium), such as memory, that stores the control program and various types of data therein. The object of the present invention is realized by a predetermined storage medium. The storage medium stores, in computer-readable manner, program codes (executable code program, intermediate code program, and source program) of the control program of the face authentication apparatus 100, which is software for realizing the aforesaid functions. The storage medium is provided to the face authentication apparatus 100. With this arrangement, the face authentication apparatus 100 (alternatively, CPU or MPU) as a computer reads out and executes program code stored in the storage medium provided.

The storage medium may be tape based, such as a magnetic tape or cassette tape; disc based, such as a magnetic disk including a floppy® disc and hard disk and optical disk including CD-ROM, MO, MD, DVD, and CD-R; card based, such as an IC card (including a memory card) and an optical card; or a semiconductor memory, such as a mask ROM, EPROM, EEPROM, and a flash ROM.

Further, the face authentication apparatus 100 may be arranged so as to be connectable to a communications network so that the program code is supplied to the face authentication apparatus 100 through the communications network. The communications network is not to be particularly limited. Examples of the communications network include the Internet, intranet, extranet, LAN, ISDN, VAN, CATV communications network, virtual private network, telephone network, mobile communications network, and satellite communications network. Further, a transmission medium that constitutes the communications network is not particularly limited. Examples of the transmission medium include (i) wired lines such as IEEE 1394, USB, power-line carrier, cable TV lines, telephone lines, and ADSL lines and (ii) wireless connections such as IrDA and remote control using infrared light, Bluetooth®, 802.11, HDR, mobile phone network, satellite connections, and terrestrial digital network. Note that the present invention can be also realized by the program codes in the form of a computer data signal embedded in a carrier wave which is embodied by electronic transmission.

### Industrial Applicability

The present invention can further improve an accuracy of the face authentication procedure without need for any special video equipment and others. Therefore, the present invention is suitably applicable to a wide variety of apparatuses including an image processing apparatus that processes facial image data of a person.

### Reference Signs List

- 10: Image data
- 20: Operation section
- 30: Display section
- 40: Control section
- 50: Face authentication section (identifying means)
- 51: Face image obtaining section (extracting means, image supplying means)
- 52: Face detecting section (extracting means)
- 53: Facial characteristics extracting section (extracting means)
- 54: Gender/age estimating section (estimating means)
- 55: Authentication information registering section
- 56: Face verifying section (extracting means)
- 57: Degree-of-similarity determining section (estimating means)
- 58: Gender/age comparing section (comparing means)
- 59: Authentication result output section (identifying means)
- 90: Storage section
- 91: Image storage section (person image storage section)
- 92: Face authentication database (registered facial information storage section)
- 93: Facial characteristics information storage section
- 94: Attribute information storage section
- 100: Face authentication apparatus (person image search system)

## Claims

1. A face authentication apparatus comprising extracting means that determines degrees of similarity between incoming input face image and a plurality of registered facial information items that have been preregistered, and then extracts the registered facial information item having the degree of similarity to the input face image of not less than a predetermined value,
the registered facial information items being respectively registered together with attribute information items on persons related to the registered facial information items,
the face authentication apparatus further comprising: identifying means that, if the extracting means extracts the plurality of registered facial information items, identifies which of the plurality of registered facial information items that have been extracted by the extracting means is the registered facial information item containing a person related to the input face image,
the identifying means comprising:
estimating means that analyzes the input face image, estimates an attribute of the person related to the input face image from a result of the analysis, and then outputs an estimated attribute information item that represents the attribute thus estimated; and
comparing means that makes comparison between the estimated attribute information item having been outputted from the estimating means and the attribute information items on the persons related to the plurality of registered facial information items,
whereby the registered facial information item containing the person related to the input face image is identified on a basis of a result of the comparison made by the comparing means.

2. The face authentication apparatus according to claim 1, wherein
the estimating means outputs the estimated attribute information item for each registered facial information item having a specific degree of similarity that is lower than the highest degree of similarity by an amount falling within a predetermined range, among the thus determined degrees of similarity of the registered facial information items having been extracted by the extracting means.

3. The face authentication apparatus according to claim 1 or 2, wherein
the estimating means outputs (i) the estimated attribute information item for a registered facial information item having the highest degree of similarity and (ii) the estimated attribute information item for a registered facial information item having a predetermined n-th highest degree of similarity, among the thus determined degrees of similarity of the registered facial information items having been extracted by the extracting means.

4. The face authentication apparatus according to any one of claims 1 through 3, wherein
each of the attribute information items includes at least one of information items on age, gender, and race.

5. The face authentication apparatus according to any one of claims 1 through 4, wherein
each of the attribute information items includes at least two of information items on age, gender, and race, and
the comparing means makes the comparison to determine if at least one match occurs in the at least two of the information items.

6. A person image search system comprising:
a face authentication apparatus according to any one of claims 1 through 5;
a person image storage section for storing person images each of which represents at least a face region of a person;
a registered facial information storage section for storing a plurality of registered facial information items;
selecting means for selecting the registered facial information item from among the plurality of registered facial information items stored in the registered facial information storage section;
image supplying means for reading the person image from the person image storage section, detecting the face region from the person image thus read, and then supplying the face region thus detected as an input face image to the face authentication apparatus;
authentication result obtaining means for obtaining a result of face authentication made for the input face image having been supplied from the image supplying means from the face authentication apparatus; and
searching means for searching, on a basis of the result having been obtained by the authentication result obtaining means, for a person image containing a person represented by the registered facial information item having been selected by the selecting means through the person images stored in the person image storage section.

7. A face authentication apparatus control program that operates a face authentication apparatus according to any one of claims 1 through 5, the face authentication apparatus control program causing a computer to function as each means in the face authentication apparatus.

8. A computer-readable recording medium storing a face authentication apparatus control program according to claim 7.

9. A method of controlling a face authentication apparatus that comprises extracting means that determines degrees of similarity between incoming input face image and a plurality of registered facial information items that have been preregistered, and then extracts the registered facial information item having the degree of similarity to the input face image of not less than a predetermined value,
the registered facial information items being respectively registered together with attribute information items on persons related to the registered facial information items,
the method comprising:
an identifying step of identifying means included in the face authentication apparatus, if the extracting means extracts the plurality of registered facial information items, identifying which of the plurality of registered facial information items thus extracted is the registered facial information item containing a person related to the input face image,
the identifying step comprising:
an estimating step of analyzing the input face image, estimating an attribute of the person related to the input face image from a result of the analysis, and then outputting an estimated attribute information item that represents the attribute thus estimated; and
a comparing step of making comparison between the attribute information item thus estimated in the estimating step and the attribute information items on the persons related to the plurality of registered facial information items,
whereby the registered facial information item containing the person related to the input face image is identified on a basis of a result of the comparison made in the comparing step.
